# EUROPEAN PATENT SPECIFICATION

(11) **EP 2 704 597 B1**
(45) Date of publication and mention of the grant of the patent: **07.04.2021**
(21) Application number: 12732703.9
(22) Date of filing: 04.05.2012
(51) Int. Cl.: A23K 50/20, A23K 20/163, A61P 43/00

(54) **PERFORMANCE ENHANCING COMPOSITIONS AND METHODS OF USE**
LEISTUNGSSTEIGERNDE ZUSAMMENSETZUNGEN UND VERWENDUNGSVERFAHREN DAFÜR
COMPOSITIONS AMÉLIORANT L'EFFICACITÉ, ET PROCÉDÉS D'UTILISATION

(30) Priority: 05.05.2011 US 201161482655 P
(43) Date of publication of application: 12.03.2014
(73) Proprietor: Life Science Nutrition AS, 6160 Hovdebygda (NO)
(72) Inventor: REMMEREIT, Jan, N-6100 Volda (NO)
(74) Representative: Algemeen Octrooi- en Merkenbureau B.V.
(86) International application number: PCT/IB2012/001092
(87) International publication number: WO 2012/150504

(56) References cited:
- EP-A1- 2 258 217
- EP-B1- 1 242 436
- WO-A1-2006/020210
- WO-A1-2009/000803
- WO-A1-2010/009474
- WO-A1-2010/120682
- US-A1- 2003 129 278
- DATABASE FSTA [Online] INTERNATIONAL FOOD INFORMATION SERVICE (IFIS), FRANkFURT-MAIN, DE; BRZESKI E ET AL: "Levels of N-acetylneuraminic acid and fat in milk of Arab mares. (translated)", Database accession no. FS-1980-05-P-0955 & ROCKINE NAUKOWE ZOOTECHNII, vol. 6, no. 1, 1979, page 29, ROCZNIKI NAUKOWE ZOOTECHNIKI 1979 INST. HODOWLI I TECH. PRODUKCJI ZWIERZECEJ, AR, KRAKOW, POLAND
- DATABASE FSTA [Online] INTERNATIONAL FOOD INFORMATION SERVICE (IFIS), FRANkFURT-MAIN, DE; MARTUZZI F ET AL: "Macro- and micromineral elements of the milk and sialic acid bound to casein and to whey proteins in nursing mares of Bardigiano horse breed.", Database accession no. FS-2000-08-Pe1240 & ANNALI DELLA FACOLTA DI MEDICINA VETERINARIA, vol. 18, 1998, page 57, ANNALI DELLA FACOLTA DI MEDICINA VETERINARIA, PARMA 1998, PUBL ISTITUTO DI ZOOTEC., ALIMENTAZIONE E NUTR., FAC. DI MED. VET., UNIV. DEGLI STUDI DI PARMA, ITALY

## Description

### FIELD OF THE INVENTION

The present invention relates to a method of improving a performance parameter in a horse, wherein said performance parameter is chosen from increase in lean muscle mass, reduction in lactic acid, increase in workload tolerance, and faster recuperation after workload, wherein said method comprises administering to said horse a composition formulated to provide 0.1 to 10 mg/150 kg subject/day of n-glycolylneuraminic acid.

### BACKGROUND OF THE INVENTION

The American Horse Council, The Food and Agriculture Organization of the United Nations and the European Union estimate the worldwide population of horses to be in the area of 60-65 million. The current European population is estimated to 7-8 million horses, with approximately 3.9M horses in Great Britain, Germany, and France. The current North America population is estimated to 12-13 million horses, with 9-10 million horses in the United States and 2-3 million horses in Canada. South America is estimated to have 15-16 million horses and the Asia/Pacific rim population is estimated to 22-23 million horses, with China having 12-13 million horses.

Britain's equine Industry has a combined economic impact of seven billion pounds, over half of which derives from horseracing. The equine industry as a whole provides direct and indirect employment to between 220 - 270,000 people. Horseracing is Britain's second most popular spectator sport, with over six million racing spectators each year. The UK has a world leading equine veterinary profession with 17,260 registered practicing vets. The total spent on equine healthcare per annum is £ 344 million, of which £ 254 million is veterinary fees. The horse industry in the US contributes $ 39 billion in direct economic impact to the US economy. When indirect and induced spending are included, the industry's economic impact reaches $ 102 billion.

As can be seen, substantial financial resources are devoted to ensuring the health and well being of horses. Likewise, the horse racing industry has a substantial economic impact. Accordingly, there is great need for supplements which enhance the performance of horses, as well as which improve the health and well being of horses.

Several benefits have been attributed to sialic acid, its precursors or derivatives. For example WO2006020210 describes n-glycol neuramamic acid and its use in improving the performance and wellbeing of a sports person, WO2010120682 describes the use of sialic acid compositions for improving wellbeing by alleviating infection related complaints, EP2258217 describes the use of sialic acid and/or its precursors for improving infant learning and development and WO2009000803 describes sialic acid derivatives for improving gastrointestinal health EP1242436 also teaches that compositions containing sialic acid have gastro-intestinal benefits, and these may be derived from mare's milk.

### SUMMARY OF THE INVENTION

Method of improving a performance parameter in a horse, wherein said performance parameter is chosen from increase in lean muscle mass, reduction in lactic acid, increase in workload tolerance, and faster recuperation after workload, wherein said method comprises administering to said horse a composition formulated to provide 0.1 to 10 mg/150 kg subject/day of n-glycolylneuraminic acid.

In some aspects said n-glycolylneuraminic acid is derived from natural sources.

In some aspects said natural source is a non-animal or non-mammalian source.

In some aspects said n-glycolylneuraminic acid is chemically synthesized.

In some aspects the method according to the invention further comprises administering at least one composition selected from the group consisting of Avena sativa extract, Fabaceae extract, and Holothuroidea echinodermata extract.

In some aspects the composition further comprises one or more mineral supplements selected from the group consisting of Fe, Zn, Cu and Mn.

In some aspects said composition is formulated to provide a daily dosage of said n-glycolylneuraminic acid, selected from the group consisting of 0.3 to 5 mg/150 kg subject/day, and 0.5 to 2 mg/150 kg subject/day.

In some aspects said composition is formulated as a powder, tablet, bolus, liquid or gel.

In some aspects said composition is formulated for an administration mode selected from the group consisting of enteral administration, parenteral administration, oral administration, subcutaneous administration, intramuscular administration, and intravenous administration.

### DESCRIPTION OF THE FIGURES

**Figure 1****:** Body mass increases by 3 % after 8 months feed on GRPA. (p=0.006)
**Figure 2****:** Lactic acid decreases by 22 % after 8 months GRPA feed (p=0.002)
**Figure 3****:** GRPA decreases the blood level of lactic acid significantly after hill-test. (12.6 % reduction, p=0.0017) (Lactic acid measured with Lactate Pro)
**Figure 4****:** GRPA reduces the blood level of CK significantly after 8 months (15% reduction, p=0.014).
**Figure 5****: GRPA** reduces CK value significantly after conducted hill-test (12.6% reduction, p=0.029)
**Figure 6****:** GRPA reduces the number of joint injections and flu/cold significantly after 8 months (p=0.00006 and p=0.00004).

### DEFINITIONS

As used herein, "sialic acid" is a generic term for the N- or O-substituted derivatives of neuraminic acid, a monosaccharide with a nine-carbon backbone. It is also the name for the most common member of this group, N-acetylneuraminic acid (Neu5Ac or NANA). Sialic acids are found widely distributed in animal tissues and to a lesser extent in other species ranging from plants and fungi to yeasts and bacteria, mostly in glycoproteins and gangliosides. The amino group generally bears either an acetyl or glycolyl group but other modifications have been described. The hydroxyl substituents may vary considerably: acetyl, lactyl, methyl, sulfate, and phosphate groups have been found.

As used herein, the term "sialic acid precursor" refers to a compound that is used in the biosynthesis of a sialic acid.

As used herein, the term "extract" refers to a substance made by extracting a part of a raw material.

As used herein, the term "phytonutrient" refers to organic compounds isolated from plants that have a biological effect, and includes, but is not limited to, compounds of the following classes: isoflavonoids, oligomeric proanthcyanidins, indol-3-carbinol, sulforaphone, fibrous ligands, plant phytosterols, ferulic acid, anthocyanocides, triterpenes, omega 3/6 fatty acids, polyacetylene, quinones, terpenes, cathechins, gallates, and quercitin.

As used herein, the term "functional foods" refers to food products that include biologically active nutraceutical agents.

As used herein, the terms "nutraceutical agent," and related terms, refer to natural, bioactive chemical compounds that have health promoting, disease preventing or medicinal properties. Examples of nutraceutical agents include, but are not limited to, Allium Cepa, Allium Sativum, Aloe Vera, Angelica Species, Naturally Occurring Antioxidants, Aspergillus Oryzae Enzyme Therapy, barley grass, Bromelain, Carnitine, Carotenoids and Flavonoids, Catechin, Centella Asiatica (Gotu kola), Coenzyme Q10, Chinese Prepared Medicines, Coleus Forskohlii, Commiphora Mukul, Crataegus Oxyacantha (Hawthorne), Curcuma Longa (Turmeric), Echinacea Species (Purple Coneflower), Eleutherococcus Senticosus (Siberian Ginseng), Ephedra Species, Dietary Fish Oil Consumption and Fish Oil Supplementation, Genistein, Ginkgo Biloba, Glycyrrhiza (Licorice), Hypericum Perforatum (St. John's Wort), Hydrastis (Goldenseal) and Other Berberine-Containing Plants, Lactobacillus, Lobelia (Indian Tobacco), Melaleuca Alternifolia, Mentha Piperita, NGNA, Panax Ginseng, Pancreatic Enzymes, Piper Mythisticum, Procyanidolic Oligomers, Pygeum Africanum, Quercetin, Sarsaparilla Species, Serenoa Repens (Saw palmetto, Sabal serrulata), Silybum Marianum (Milk Thistle), Rosemary/Lemon balm, Selenite, Tabebuia Avellanedae (LaPacho), Taraxacum Officinale, Tanacetum Parthenium (Feverfew), Taxol, Uva Ursi (Bearberry), Vaccinium Myrtillus (Blueberry), Valerian Officinalis, Viscum Album (Mistletoe), Vitamin A, Beta-Carotene and Other Carotenoids, and Zingiber Officinale(Ginger).

As used herein, the terms "subject" and "patient" refer to any animal, such as a mammal (e.g., human, horse, dog, cat, bovine, swine, and sheep), bird, or fish.

As used herein, the term "physiologically acceptable carrier" refers to any carrier or excipient commonly used with pharmaceuticals. Such carriers or excipients include, but are not limited to, oils, starch, sucrose and lactose.

As used herein, the term "oral delivery vehicle" refers to any means of delivering a pharmaceutical orally, including, but not limited to, capsules, pills, tablets and syrups.

As used herein, the term "food product" refers to any food or feed suitable for consumption by humans, non-ruminant animals, or ruminant animals. The "food product" may be a prepared and packaged food (*e*.*g*., mayonnaise, salad dressing, bread, or cheese food) or an animal feed (*e*.*g*., extruded and pelleted animal feed or coarse mixed feed). "Prepared food product" means any pre-packaged food approved for human consumption.

As used herein, the term "foodstuff" refers to any substance fit for human or animal consumption.

As used herein, the term "dietary supplement" refers to a small amount of a compound for supplementation of a human or animal diet packaged in single or multiple does units. Dietary supplements do not generally provide significant amounts of calories but may contain other micronutrients (e.g., vitamins or minerals).

As used herein, the term "nutritional supplement" refers to a composition comprising a "dietary supplement" in combination with a source of calories. In some embodiments, nutritional supplements are meal replacements or supplements (e.g., nutrient or energy bars or nutrient beverages or concentrates).

### DETAILED DESCRIPTION OF THE INVENTION

The present invention relates to a method of improving a performance parameter in a horse, wherein said performance parameter is chosen from increase in lean muscle mass, reduction in lactic acid, increase in workload tolerance, and faster recuperation after workload, wherein said method comprises administering to said horse a composition formulated to provide 0.1 to 10 mg/150 kg subject/day of n-glycolylneuraminic acid. The disclosure further relates to a composition containing sialic acids or precursors, and their use to improve performance-related parameters in mammals, particularly horses. Accordingly, in some embodiments, the present disclosure provides compositions comprising an effective amount of a sialic acid or sialic precursor.

The use of a variety of sialic acids is contemplated in the disclosure. Examples include, but are not limited to, N- or O-substituted derivatives of neuraminic acid, and in particularly preferred embodiments are n-glycolylneuraminic acid, n-acetylneuraminic acid. Likewise, the use of a variety of sialic precursors is contemplated in the disclosure, including but not limited to, N-Acetyl-D-mannosamine. In some preferred aspects, the sialic acid or sialic acid precursor have a purity selected from the group consisting of greater than 90%, 95%, 99%, and 99.5% pure. The use of sialic acids and precursors from a variety sources is contemplated. In some aspects, the sialic acid or sialic acid precursor are derived from natural sources. In some aspects, the natural source is a non-animal source. In some aspects, the sialic acid or sialic acid precursor are chemically synthesized.

NGNA compositions of the present invention may be delivered in any suitable format. In some embodiments, NGNA is provided from sea cucumbers, e.g., an extract of sea cucumbers, or is prepared from chitin. In some embodiments, the NGNA is preferably about greater than 90%, 95%, 99% or 99.9% pure. In some embodiments, the NGNA is HPLC purified. In some embodiments, NGNA is prepared as described in WO 00/38967. For example, N-glycolylneuraminic acid can be purchased commercially from, for example, Sigma Chemical Company, St. Louis, MO. N-glycolylneuraminic acid also can be synthesized. For example, CMP-N-acetylneuraminic acid hydroxylase can be used to synthesize N-glycolylneurarninic acid as its CMP-glycoside. See, Schlenzka et al., Glycobiolog, 1994, 4(5):675-683. Non-enzymatic methods of synthesis include, for example, synthesis from N- acetylneuraminic acid using methanol or hydrochloric acid and benzylalcohol. Other synthesis methods are described in Choi et al., J. Org. Chem., 1996, 61:8/39 (from mannosamine), Faillard et al., J. Physiol. Chem.' 1965, 344:167 (from glucosamine), U.S. Patent No. 4,774,326 and U.S. Patent No.4,774,327.

In some aspects of the disclosure, compositions comprising a combination of sialic acids and sialic acid precursors are provided. In some aspects, the compositions comprise at least two isolated and purified sialic acids or sialic acid precursors. In some aspects, the at least two sialic acids or sialic acid precursors have a purity selected from the group consisting of greater than 90%, 95%, 99%, and 99.5% pure. In some aspects, the at least two sialic acids or sialic acid precursors are selected from the group consisting of n-glycolylneuraminic acid, n-acetylneuraminic acid and N-Acetyl-D-mannosamine. In some aspects, the composition may comprise two or more of the following sialic acids: Neuraminic acid, 5-N-Acetyl-4-O-acetyl-neuraminic acid, 5-N-Acetyl-7-O-acetyl-neuraminic acid, 5-N-Acetyl-8-O-acetyl-neuraminic acid, 5-N-Acetyl-9-0-acetyl-neuraminic acid, 5-N-Acetyl-4,9-di-O-acetyl-neuraminic acid, 5-N-Acetyl-7,9-di-O-acetyl-neuraminic acid, 5-N-Acetyl-8,9-di-O-acetyl-neuraminic acid, 5-N-Acetyl-7,8,9-tri-O-acetyl-neuraminic acid, 5-N-Acetyl-9-O-L-lactyl-acetyl-neuraminic acid, 5-N-Acetyl-4-O-acetyl-9-O-lactyl-acetyl-neuraminic acid, 5-N-Acetyl-8-O-methyl-neuraminic acid, 5-N-Acetyl-9-O-acetyl-8-O-methyl-neuraminic acid, 5-N-Acetyl-8-0-sulpho-neuraminic acid, 5-N-Acetyl-9-O-phosphoro-neuraminic acid, 5-N-Acetyl-2-deoxy-2,3-didehydro-neuraminic acid, 5-N-Acetyl-9-O-acetyl-2-deoxy-2,3-didehydro-neuraminic acid, 5-N-Acetyl-2-deoxy-2,3-didehydro-9-O-lactyl-neuraminic acid, 5-N-Acetyl-2,7-anhydro-neuraminic acid, 4-0-Acetyl-5-N-glycolyl-neuraminic acid, 7-O-Acetyl-5-N-glycolyl-neuraminic acid, 8-0-Acetyl-5-N-glycolyl-neuraminic acid, 9-0-Acetyl-5-N-glycolyl-neuraminic acid, 7,9-Di-O-acetyl-5-N-glycolyl-neuraminic acid, 8,9-Di-O-acetyl-5-N-glycolyl-neuraminic acid, 7,8,9-Tri-O-acetyl-5-N-glycolyl-neuraminic acid, 5-N-glycolyl-9-O-lactyl-neuraminic acid, 5-N-glycolyl-8-O-methyl-neuraminic acid, 9-0-Acetyl-5-N-glycolyl-8-O-methyl-neuraminic acid, 7,9-di-O-Acetyl-5-N-glycolyl-8-0-methyl-neuraminic acid, 5-N-glycolyl-8-0-sulpho-neuraminic acid, N-(O-acetyl)glycolylneuraminic acid, N-(O-Methyl)glycolylneuraminic acid, 2-Deoxy-2,3-didehydro-5-N-glycolylneuraminic acid, 9-O-Acetyl-2-deoxy-2,3-didehydo-5-N-glycolyl-neuraminic acid, 2-Deoxy-2,3-didehydro-5-N-glycolyl-9-O-lactyl-neuraminic acid, 2-Deoxy-2,3-didehydro-5-N-glycolyl-8-O-methyl-neuraminic acid, 2,7-Anhydro-5-N-glycolyl-neuraminic acid, 2,7-Anhydro-5-N-glycolyl-8-0-methyl-neuraminic acid, 2-Keto-3-deoxynononic acid, and 9-O-Acetyl-2-keto-3-deoxynononic acid.

In some aspects, the first sialic acid or precursor and second sialic acid precursor are provided at a ratio of about 20:1 to 1:20, 10:1 to 1:10, 5:1 to 1:5, 2:1 to 1:2, 20:1 to 10:1, 10:1 to 5:1, 5:1 to 2:1, 2:1 to 1:1. In some embodiments, the first sialic acid or precursor may be one of n-glycolylneuraminic acid, n-acetylneuraminic acid and N-Acetyl-D-mannosamine and the second sialic acid or precursor may be another of n-glycolylneuraminic acid, n-acetylneuraminic acid and N-Acetyl-D-mannosamine. In some embodiments, the composition may comprise three or more sialic acids or precursors.

In some aspects, the present disclosure provides compositions or formulations that are formulated to provide a daily dosage of the sialic acid or precursor (or combination thereof) selected from the group consisting of 1 microgram to 100 mg/day, 100 microgram to 20 mg/day, and 200 microgram to 10 mg/day. In some aspects, the composition is formulated to provide a daily dosage of the sialic acid or precursor of 0.1 to 10 mg/150 kg subject/day, 0.3 to 5 mg/150 kg subject/day, and 0.5 to 2 mg/150 kg subject/day. In some aspects, an effective amount comprises the daily dosages just described.

In some embodiments of the invention, the compositions further comprise one or more extracts. In some embodiments, the extract is selected from the group consisting of an Avena sativa extract, a Fabaceae extract, and and a Holothuroidea echinodermata extract and combinations thereof. In some embodiments, the extracts are aqueous extracts, while in other embodiments, the extract is an ethanol extract, a super critical fluid extract, or an organic solvent extract. Oats (Avena sativa) is a species of cereal grain grown for its seed and rich on vitamin B and several minerals. One common use is as livestock feed and oats make up a part of the daily diet of horses, up to about 20 percent of daily intake. Fabaceae or Leguminosae is the third largest family of flowering plants, which is commonly known as the legume family. Grain legumes are cultivated for their seeds, which are used for human and animal consumption. Grain legumes include herbaceous plants like beans, lentils, lupines, peas and peanuts. Holothuroidea echinodermata is the sea cucumber. The compositions of the present invention preferably comprise one or more extracts from these sources.

In some embodiments of the invention, the compositions of the present invention further comprise one or more minerals or salts or derivatives thereof, in known effective amounts for daily supplementation for the desired subject. Examples of such minerals include Fe, Zn, Cu and Mn.

In some aspects of the disclosure, the composition further comprise one or more phytonutrients, nutraceutical agents, or fatty acids. Examples of suitable phytonutrients and nutraceutical agents include, but are not limited to, carotenoids, flavonoids (including isoflavonoids and neo flavonoids; e.g., Luteolin, Apigenin, Tangeritin, Quercetin, Kaempferol, Myricetin, Fisetin, Isorhamnetin, Pachypodol, Rhamnazin, Hesperetin, Naringenin, Eriodictyol, Homoeriodictyol, Taxifolin (or Dihydroquercetin), Dihydrokaempferol, Genistein, Daidzein, Glycitein, Catechins (Catechin (C), Gallocatechin (GC), Catechin 3-gallate (Cg), Gallocatechin 3-gallate (GCg)), Epicatechins (Epicatechin (EC), Epigallocatechin (EGC), Epicatechin 3-gallate (ECg), Epigallocatechin 3-gallate (EGCg)), Cyanidin, Delphinidin, Malvidin, Pelargonidin, Peonidin, Petunidin), resveratrol, Coenzyme Q10, B6/pyridoxamine, n-acetyl cysteine, phosphatidyl serine, Vitamin E, Vitamin B6 (pyridoxine), Gingko biloba extract (preferably at least 20% ginkgolides).

The compositions of the present disclosure may be provided in a variety of formulations. In preferred aspects, the formulations provide the preferred daily dosage of the sialic acid or precursor. In some embodiments, the compositions administered according to the method of the invention are formulated for an administration mode selected from the group consisting of enteral administration, parenteral administration, oral administration, subcutaneous administration, intramuscular administration, and intravenous administration.

In some aspects, the present disclosure provides oral dosage forms comprising sialic acid compositions as described above. The ingredients of the oral dosage form of this invention are preferably contained in acceptable excipients and/or carriers for oral consumption. The actual form of the carrier, and thus, the oral dosage form itself, is not critical. The carrier may be a liquid, gel, gel cap, capsule, powder, solid tablet (coated or non-coated), tea, or the like. The oral dosage form is preferably in the form of a tablet or capsule such as a hard gelatin capsule. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. Further details on techniques for formulation for and administration may be found in the latest edition of Remington 's Pharmaceutical Sciences (Maack Publishing Co., Easton, Pa.).

In some aspects of the disclosure, the sialic acid composition is provided in a shot format comprising a daily dosage as described above in an aqueous base of from about 10 to 100 ml, preferably 10 to 80 ml, more preferably about 10 to 60 ml and most preferably about 10 to 50 ml. The shots may preferably comprise one or more of the additional dietary supplements or vitamins listed above and a flavoring agent.

In other embodiments, the compositions administered according to the method of the invention are provided as a powder or liquid suitable for adding by the consumer to a feed, food, beverage or water. For example, in some embodiments, the dietary supplement can be administered to a subject in the form of a powder, for instance to be used by mixing into water, or by adding to a feed ration, for example an equine feed ration. In some embodiments, the compositions are provided for use as a top-dressing which can be added to or mixed with a feed ration. In these embodiments, the compositions are formulated to provide the preferred daily dosages of sialic acids or precursors when added to the feed ration. As a non-limiting example, the compositions may be provided as a powder in which ten grams of the powder contains 1 mg of sialic acid or precursor. The powder may then be added to the daily feed ration of horse at a rate of 10 grams per 150 kg of the weight of the horse (i.e., a horse weighing 450 kg would receive 30 grams of powder per day).

The compositions of the present disclosure may also contain optional ingredients including, for example, herbs, vitamins, minerals, enhancers, colorants, sweeteners, flavorants, inert ingredients, and the like. In some particularly preferred aspect, the ingredient is suitable for feeding to a horse, for example, molasses, apple flavoring, cane sugar, and the like.

In some aspects, the compositions further comprise vitamins and minerals including, but not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D₃; cyanocobalamin; sodium selenite; copper sulfate; vitamin A; vitamin C; inositol; potassium iodide.

Many different feed rations may be formulated for animals from many different feed ingredients. Rations are generally formulated to provide-nutrients in accordance with National Research Council standards. The feedstuffs used in the ration are chosen according to market price and availability. Thus, some components of the ration may change over time. In the feeds of the present invention, the ration will preferably contain compositions described above in an amount sufficient to deliver the preferred daily dosage, but other components may vary over time based on the price of the component. For discussions on feed ration formulation, actual rations and NRC guidelines, see Church, Livestock Feeds and Feeding, O&B Books, Inc., Corvallis OR (1984) and Feeds and Nutrition Digest, Ensminger, Oldfield and Heineman eds., Ensminger Publishing Corporation, Clovis, Calif. (1990).

The animal feed rations of the present disclosure may be characterized according to NRC requirements. NRC requirements may be found in Church, Livestock Feeds and Feeding, O&B Books, Inc., Corvallis Oreg. (1984), or other nutritional standards. Equine and other animal rations are traditionally balanced using the protein and energy requirements, and then adjusted if needed to meet the other requirements. The equine feeds of the present invention will contain sialic acids or precursors in amounts sufficient to deliver the preferred daily dosages, plus other feed materials necessary to balance the feed to meet the NRC requirements for the different stages of growth and maintenance.

The relative amounts of protein and energy are preferably adjusted to reflect Nutritional Standards requirements or requirement of a trainer, for example a trainer or race or working horses. The amounts of feed components will vary with the stage of animal fed. A growing ration for young animals will have higher protein levels, while a finishing ration for finishing animals for market will have higher energy values which are supplied by carbohydrates.

In a preferred aspect, the compositions described above are incorporated into a pelleted or textured feed for administration to horses. Pelleted feed is created by first mixing feed components and then compacting and extruding the feed components through a die with heat and pressure. The feed is pelleted by methods known in the art, which are described in MacBain, Pelleting Animal Feed, American Feed Manufacturers Association, Arlington, Va. (1974).

In an aspect of the disclosure, rations containing the foregoing compositions may also be formulated for animals other than horses. The amount of CFAP administered is not critical as long as it is enough to be effective in decreasing body fat and increasing feed efficiency or other benefits. The feeds are formulated as above, and tailored to the requirements of the animal to be fed in accordance with NRC guidelines. For example, feeds may be formulated for dogs, cats, poultry, sheep and cattle. Various rations are given in the examples. Various feed formulations, balancing methods and requirements for these animals are discussed in Church, Livestock Feeds and Feeding, O&B Books, Inc., Corvallis Oreg. (1984) and Feeds and Nutrition Digest, Ensminger, Oldfield and Heineman eds., Ensminger Publishing Corporation, Clovis, Calif. (1990). It should be borne in mind that feed companies and livestock producers develop their own nutritional requirements based on historical results. The present disclosure makes the use of CFAP possible in these various feeds because the CFAP may be produced economically in bulk quantities.

In other aspects, the present disclosure provides nutritional supplements (e.g., energy bars or meal replacement bars or beverages) comprising the foregoing compositions. The nutritional supplement may serve as meal or snack replacement and generally provide nutrient calories. Preferably, the nutritional supplements provide carbohydrates, proteins, and fats in balanced amounts. The nutritional supplement can further comprise carbohydrate, simple, medium chain length, or polysaccharides, or a combination thereof. A simple sugar can be chosen for desirable organoleptic properties. Uncooked cornstarch is one example of a complex carbohydrate. If it is desired that it should maintain its high molecular weight structure, it should be included only in food formulations or portions thereof which are not cooked or heat processed since the heat will break down the complex carbohydrate into simple carbohydrates, wherein simple carbohydrates are mono- or disaccharides. The nutritional supplement contains, in one embodiment, combinations of sources of carbohydrate of three levels of chain length (simple, medium and complex; e.g., sucrose, maltodextrins, and uncooked cornstarch).

Sources of protein to be incorporated into the nutritional supplement of the invention can be any suitable protein utilized in nutritional formulations and can include whey protein, whey protein concentrate, whey powder, egg, soy flour, soy milk soy protein, soy protein isolate, caseinate (e.g., sodium caseinate, sodium calcium caseinate, calcium caseinate, potassium caseinate), animal and vegetable protein and mixtures thereof. When choosing a protein source, the biological value of the protein should be considered first, with the highest biological values being found in caseinate, whey, lactalbumin, egg albumin and whole egg proteins. In a preferred aspect, the protein is a combination of whey protein concentrate and calcium caseinate. These proteins have high biological value; that is, they have a high proportion of the essential amino acids. See Modern Nutrition in Health and Disease, eighth edition, Lea & Febiger, publishers, 1986, especially Volume 1, pages 30-32.

The nutritional supplement can also contain other ingredients, such as one or a combination of other vitamins, minerals, antioxidants, fiber and other dietary supplements (e.g., protein, amino acids, choline, lecithin, omega-3 fatty acids). Selection of one or several of these ingredients is a matter of formulation, design, consumer preference and end-user. The amounts of these ingredients added to the dietary supplements of this invention are readily known to the skilled artisan. Guidance to such amounts can be provided by the U.S. RDA doses for children and adults. Further vitamins and minerals that can be added include, but are not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D₃; cyanocobalamin; sodium selenite; copper sulfate; vitamin A; vitamin C; inositol; potassium iodide.

Flavors, coloring agents, spices, nuts and the like can be incorporated into the product. Flavorings can be in the form of flavored extracts, volatile oils, chocolate flavorings, peanut butter flavoring, cookie crumbs, crisp rice, vanilla or any commercially available flavoring. Examples of useful flavoring include, but are not limited to, pure anise extract, imitation banana extract, imitation cherry extract, chocolate extract, pure lemon extract, pure orange extract, pure peppermint extract, imitation pineapple extract, imitation rum extract, imitation strawberry extract, or pure vanilla extract; or volatile oils, such as balm oil, bay oil, bergamot oil, cedarwood oil, walnut oil, cherry oil, cinnamon oil, clove oil, or peppermint oil; peanut butter, chocolate flavoring, vanilla cookie crumb, butterscotch or toffee. In one embodiment, the dietary supplement contains cocoa or chocolate.

Emulsifiers may be added for stability of the final product. Examples of suitable emulsifiers include, but are not limited to, lecithin (e.g., from egg or soy), and/or mono- and di-glycerides. Other emulsifiers are readily apparent to the skilled artisan and selection of suitable emulsifier(s) will depend, in part, upon the formulation and final product.

Preservatives may also be added to the nutritional supplement to extend product shelf life. Preferably, preservatives such as potassium sorbate, sodium sorbate, potassium benzoate, sodium benzoate or calcium disodium EDTA are used.

In addition to the carbohydrates described above, the nutritional supplement can contain natural or artificial (preferably low calorie) sweeteners, e.g., saccharides, cyclamates, aspartamine, aspartame, acesulfame K, and/or sorbitol. Such artificial sweeteners can be desirable if the nutritional supplement is intended to be consumed by an overweight or obese individual, or an individual with type II diabetes who is prone to hyperglycemia.

The nutritional supplement can be provided in a variety of forms, and by a variety of production methods. In a preferred aspect of the disclosure, to manufacture a food bar, the liquid ingredients are cooked; the dry ingredients are added with the liquid ingredients in a mixer and mixed until the dough phase is reached; the dough is put into an extruder, and extruded; the extruded dough is cut into appropriate lengths; and the product is cooled. The bars may contain other nutrients and fillers to enhance taste, in addition to the ingredients specifically listed herein.

Servings of the nutritional supplement of the disclosure preferably contain between 0.1 g and 10.0 g of NGNA, preferably between 0.5 and 2.0 g NGNA, and even more preferably approximately 1.0 g NGNA. It is understood by those of skill in the art that other ingredients can be added to those described herein, for example, fillers, emulsifiers, preservatives, etc. for the processing or manufacture of a nutritional supplement.

In still further aspects, the present disclosure provides functional foods, including food products, prepared food products, or foodstuffs comprising nutraceutical agents. For example, in some aspects, beverages and solid or semi-solid foods comprising nutraceutical agents are provided. These forms can include, but are not limited to, beverages (e.g., water, soft drinks, milk and other dairy drinks, and diet drinks), baked goods, puddings, dairy products, confections, snack foods, or frozen confections or novelties (e.g., ice cream, milk shakes), prepared frozen meals, candy, snack products (e.g., chips), soups, spreads, sauces, salad dressings, prepared meat products, cheese, yogurt and any other fat or oil containing foods, and food ingredients (e.g., wheat flour).

The compositions according to the disclosure described above are useful for improving performance parameters as well as increasing overall health in animals, including mammals (e.g., human, horse, dog, cat, bovine, swine, and sheep), birds, and fish (i.e., subjects). In some aspects, the compositions are provided to a subject in amount sufficient to cause one or more of the following effects: reduction in injuries, reduction in infectious disease, increase in lean muscle mass, increase in oxygen uptake, reduction in lactic acid, increase in workload tolerance, improved joint and ligament health, improved behavior, increase in endurance, faster recuperation after workload, faster recuperation from disease, improved pulse parameters, improved gastric health, improved food utilization, improved appearance, improved muscularity, improved coat quality, and improved kidney function. In some embodiments, the compositions are included in a diet or used to supplement a subject to ameliorate the side effects or toxic effects of an agent that is being supplied to the subject, for example, an agent that reduces one or more of the performance factors, that inhibits the immune system (e.g., a steroid), or that has an adverse or toxic effect on the kidneys.

The composition of the present disclosure are also useful in improving social parameters in animals, including mammals (e.g., human, horse, dog, cat, bovine, swine, and sheep), birds, and fish (i.e., subjects). Interestingly, it have been found that the compositions of the present disclosure, especially sialic acid compositions, improve social parameters in animals, including mammals (e.g., human, horse, dog, cat, bovine, swine, and sheep), birds, and fish. In some aspects, the social parameter is improved sociability, reduction of anxious feelings or nervous behavior, reduction of nervous feelings or nervous behavior, increased feeling of calmness, reduction of one or more symptoms associated with social anxiety disorder (e.g., fear of social situations, nervousness, avoidance of social situations, confusion, pounding heart, sweating, shaking, blushing, muscle tension, upset stomach, and diarrhea), reduction of feeling of embarrassment, improved mood, improved social behavior, decreased shyness, improved trainability, improvement in seeking of companionship, decreased anxiety prior to athletic performance.

### EXAMPLES

### Example 1

### Formulation of GRPA

| **Appearance** | **Specification Light yellow powder** | **Result Conform** |
|---|---|---|
| **Loss on drying** | **10%max.** | **6.45%** |
| **GRPA** | **78-82%** | **80%** |
| **Fe** | **2.2-2.6%** | **2.42%** |
| **Zn** | **1.6-1.9%** | **1.76%** |
| **Cu** | **1.1-1.4%** | **1.26%** |
| **Mn** | **0.6-0.7%** | **0.65%** |

GRPA is:
NGNA: 1mg/10g GRPA w/w
Avena sativa extract powder: QS
Fabaceae extract powder: QS
Holothuroidea echinodermata extract powder:QS
Metabolized Energy: >Kcal 500/kg.
Yeast and Mold: negative
E.Coli: Negative
Arsenic: 3ppm max.
Heavy Metal: 20ppm max

### Example 2

### Effects of feeding GRPA

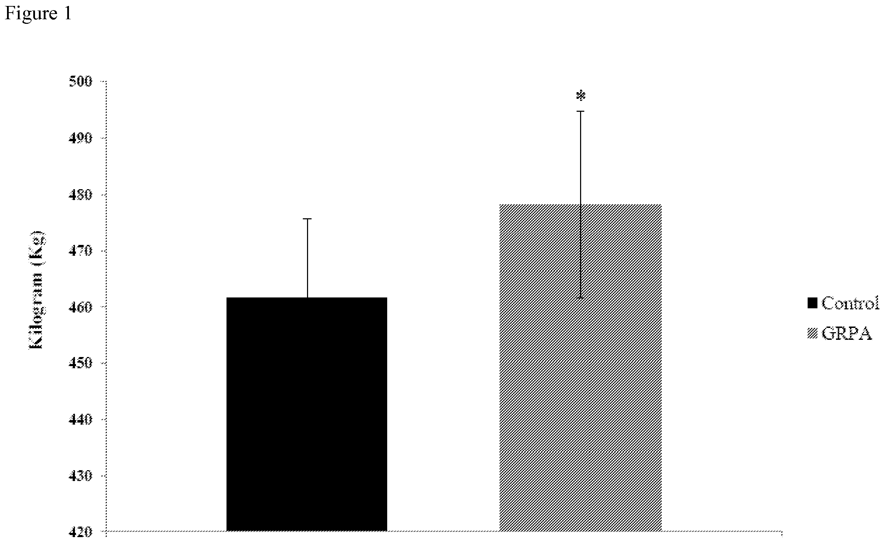

**GRPA increases muscle mass.** Increase in muscle mass is an enormous advantage for the performance level during horse race competition, increasing the ability of the horse's efficiency and speed. Upon regular training GRPA increases body weight, thus muscle mass. For results see Figure 1.

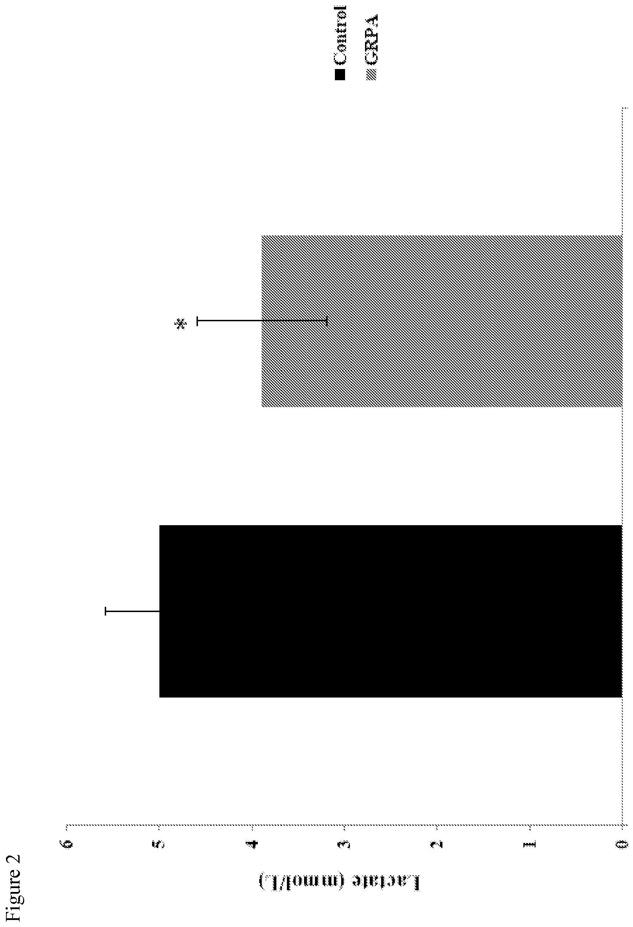

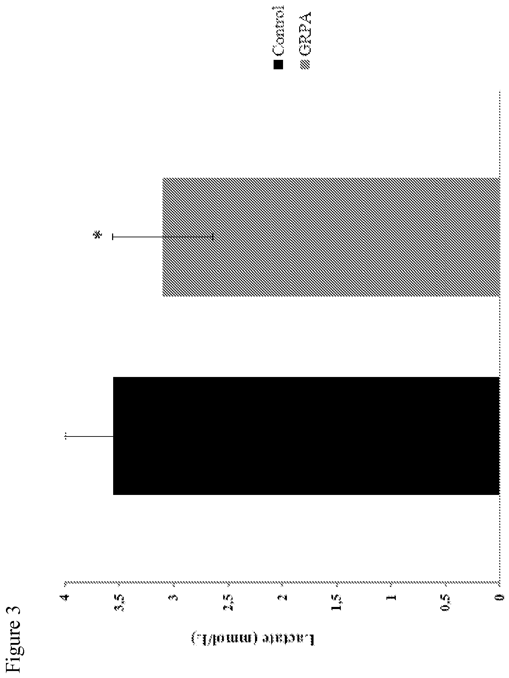

**GRPA increases muscular endurance.** With racing and heavy training the blood level of lactic acid increases, however, the level of lactic acid production depends on the horse's ability for oxygen consumption and oxygen delivery to the muscles. With high levels of lactic acid the muscle tires and decreases horses ability to keep high speed under racing. GRPA increases muscular endurance by significantly reducing the blood level of lactic acid. This gives the race horse a great advantage. Horses who feed on GRPA will have improved muscular endurance and the ability to keep high speed under racing, without tiring muscles. See Figure 2: Lactic acid decreases by 22 % after 8 months GRPA feed (p=0,002), and Figure 3: GRPA decreases the blood level of lactic acid significantly after hill-test. (12, 6 % reduction, p=0,0017) (Lactic acid measured with Lactate Pro)

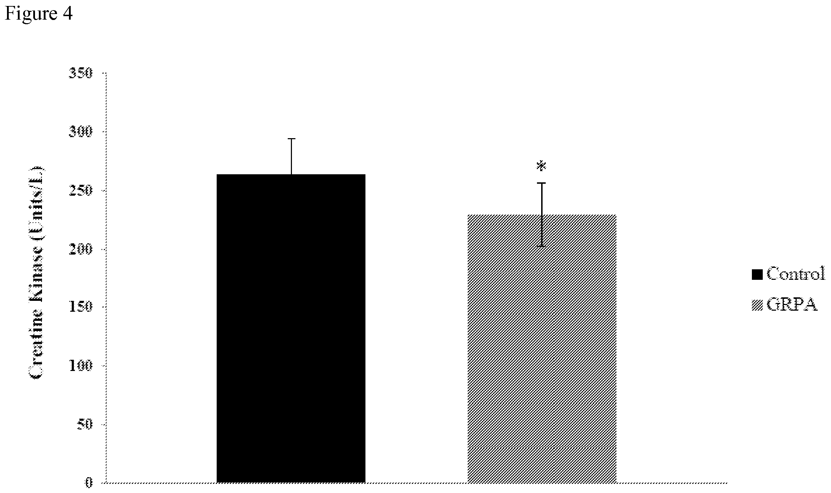

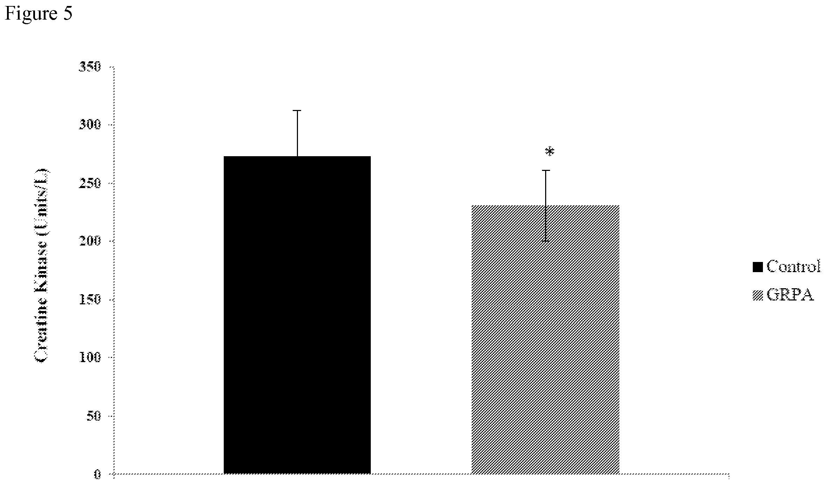

**GRPA increases workload tolerance.** After workload the horse and the muscles need time to recover. Recovery time needed depends on e.g. performed workload and the horse's ability to recover. Workload tolerance is monitored by measuring blood level of CK. Increased CK values indicates muscle breakdown, and the horse needs a longer recovery period. With GRPA, CK value is decreased with 12,6 % compared to control, resulting in reduced recovery time and increased workload tolerance, as well as increased ability to hard training without incidents of injuries. See Figure 4: GRPA reduces the blood level of CK significantly after 8 months (15% reduction, p=0,014), and Figure 5: GRPA reduces CK value significantly after conducted hill-test (12,6% reduction, p=0,029)

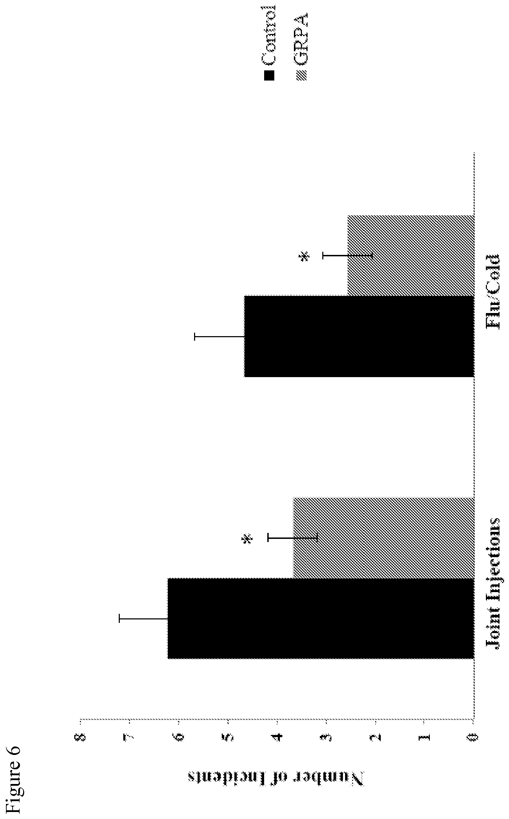

**GRPA increases the immune system.** Race horses are exposed to systematic hard workload and this increases the chance of injuries as well as incidents of general infections. The advantage of GRPA feed is that it reduces incidents of injuries with 41 % and cold/flu with 45 % compared to control group. See Figure 6: GRPA reduces the number of joint injections and flu/cold significantly after 8 months (p=0,00006 and p=0,00004)

**Concluding remarks:** GRPA gives a uniquely advantageous effect as a feed for race horses, by improving the horse's performance, growth and health, with no side effects.

### Reference Example 3

### Social effects of sialic acid supplements

A sialic acid supplement (GRPA) was provided to horses. Trainers noted improvement of social parameters in the animals fed the supplement, including generally improved sociability, improved trainability, reduced aggression, and increased contact-seeking behavior.

### Reference Example 4

### Effect of Sialic Administration in a Human Subject

A sialic supplement comprising 10 mg NANA and NGNA was consumed by a small group of human fitness enthusiasts. The preferred administration was route was either nasal or sublingual administration, which showed the most immediate effect. Oral administration was also effective, although the effects were less immediate. The subjects noted the following effects during the trial: reduced susceptibility to colds and flu, improved energy, improved mood and sociability, improved social interaction and seeking social interaction, improved feeling of well being, improved lean body mass, decreased anxiety prior to athletic performance, and improved training intensity and results.

### Reference Example 5

### Formulation of a Sialic Acid Supplement

A sialic acid supplement is formulated by combining 10 mg of n-glycolylneuraminic acid and 10 mg n-acetylneuraminic acid, each greater than 95% pure with 480 mg maltodextrin and encapsulating the mixture in a gel capsule.

### Reference Example 6

### Formulation of a Sialic Acid Supplement

A sialic acid supplement is formulated by combining 100 mg of n-glycolylneuraminic acid and 100 mg n-acetylneuraminic acid, each greater than 95% pure with 300 mg maltodextrin and encapsulating the mixture in a gel capsule.

### Reference Example 7

### Formulation of a Sialic Acid Supplement

A sialic acid supplement is formulated by combining 10 mg of n-glycolylneuraminic acid and 10 mg N-Acetyl-D-mannosamine, each greater than 95% pure with 480 mg maltodextrin and encapsulating the mixture in a gel capsule.

### Reference Example 8

### Formulation of a Sialic Acid Supplement

A sialic acid supplement is formulated by combining 100 mg of n-glycolylneuraminic acid and 100 mg N-Acetyl-D-mannosamine, each greater than 95% pure with 300 mg maltodextrin and encapsulating the mixture in a gel capsule.

### Reference Example 9

### Formulation of a Sialic Acid Supplement

A sialic acid supplement is formulated by combining 10 mg of n-acetylneuraminic acid and 10 mg N-Acetyl-D-mannosamine, each greater than 95% pure with 480 mg maltodextrin and encapsulating the mixture in a gel capsule.

### Reference Example 10

### Formulation of a Sialic Acid Supplement

A sialic acid supplement is formulated by combining 100 mg of n-acetylneuraminic acid and 100 mg N-Acetyl-D-mannosamine, each greater than 95% pure with 300 mg maltodextrin and encapsulating the mixture in a gel capsule.

### Reference Example 11

### Formulation of a Beverage Containing Sialic Acid

A beverage is formulated by dissolving 50 g of n-glycolylneuraminic acid and 50 g n-acetylneuraminic acid in 100 liters of water. A flavoring agent is added and the mixture is dispensed into 20 ml containers.

### Reference Example 12

### Formulation of a Beverage Containing Sialic Acid

A beverage is formulated by dissolving 50 g of n-glycolylneuraminic acid and 50 g N-Acetyl-D-mannosamine in 100 liters of water. A flavoring agent is added and the mixture is dispensed into 20 ml containers.

### Reference Example 13

### Formulation of a Beverage Containing Sialic Acid

A beverage is formulated by dissolving 50 g of n-acetylneuraminic acid and 50 g N-Acetyl-D-mannosamine in 100 liters of water. A flavoring agent is added and the mixture is dispensed into 20 ml containers.

### Reference Example 11

### Formulation of a Beverage Containing Sialic Acid

A beverage is formulated by dissolving 5 g of n-glycolylneuraminic acid and 5 g n-acetylneuraminic acid in 100 liters of water. The mixture is dispensed into 500 ml containers.

### Reference Example 12

### Formulation of a Beverage Containing Sialic Acid

A beverage is formulated by dissolving 5 g of n-glycolylneuraminic acid and 5 g N-Acetyl-D-mannosamine in 100 liters of water. The mixture is dispensed into 500 ml containers.

### Reference Example 13

### Formulation of a Beverage Containing Sialic Acid

A beverage is formulated by dissolving 5 g of n-acetylneuraminic acid and 5 g N-Acetyl-D-mannosamine in 100 liters of water. The mixture is dispensed into 500 ml containers.

### Reference Example 14

### Use of Sialic Acid Compositions to Improve Performance Parameters

Forty physically active adult volunteers are divided into two groups of 20. Group A receives the sialic acid formulation of Example 5 and Group B receives a placebo. The trial is conducted for a period of 12 weeks. The participants maintain existing workout and exercise patterns. At the end of the trial, Group A reports improvements, as compared to control Group B, in combinations of the following parameters: reduction in injuries, reduction in infectious disease, increase in lean muscle mass, increase in oxygen uptake, reduction in lactic acid, increase in workload tolerance, improved joint and ligament health, increase in endurance, faster recuperation after exercise, faster recuperation from disease, improved pulse parameters, improved gastric health, improved food utilization, improved appearance, improved muscularity, and improved kidney function.

### Reference Example 15

### Use of Sialic Acid Compositions to Improve Performance Parameters

Forty physically active adult volunteers are divided into two groups of 20. Group A receives the sialic acid formulation of Example 7 and Group B receives a placebo. The trial is conducted for a period of 12 weeks. The participants maintain existing workout and exercise patterns. At the end of the trial, Group A reports improvements, as compared to control Group B, in combinations of the following parameters: reduction in injuries, reduction in infectious disease, increase in lean muscle mass, increase in oxygen uptake, reduction in lactic acid, increase in workload tolerance, improved joint and ligament health, increase in endurance, faster recuperation after exercise, faster recuperation from disease, improved pulse parameters, improved gastric health, improved food utilization, improved appearance, improved muscularity, and improved kidney function.

### Reference Example 16

### Use of Sialic Acid Compositions to Improve Social Parameters

Forty adult volunteers are divided into two groups of 20. Group A receives the sialic acid formulation of Example 5 and Group B receives a placebo. The trial is conducted for a period of 12 weeks. At the end of the trial, Group A reports improvements, as compared to control Group B, in combinations of the following parameters: improved sociability, reduction of anxious feelings or nervous behavior, reduction of nervous feelings or nervous behavior, increased feeling of calmness, reduction of one or more symptoms associated with social anxiety disorder (e.g., fear of social situations, nervousness, avoidance of social situations, confusion, pounding heart, sweating, shaking, blushing, muscle tension, upset stomach, and diarrhea), reduction of feeling of embarrassment, improved mood, improved social behavior, decreased shyness, improved trainability, improvement in seeking of companionship.

### Reference Example 17

### Use of Sialic Acid Compositions to Improve Social Parameters

Forty adult volunteers are divided into two groups of 20. Group A receives the sialic acid formulation of Example 7 and Group B receives a placebo. The trial is conducted for a period of 12 weeks. At the end of the trial, Group A reports improvements, as compared to control Group B, in combinations of the following parameters: improved sociability, reduction of anxious feelings or nervous behavior, reduction of nervous feelings or nervous behavior, increased feeling of calmness, reduction of one or more symptoms associated with social anxiety disorder (e.g., fear of social situations, nervousness, avoidance of social situations, confusion, pounding heart, sweating, shaking, blushing, muscle tension, upset stomach, and diarrhea), reduction of feeling of embarrassment, improved mood, improved social behavior, decreased shyness, improved trainability, improvement in seeking of companionship.

## Claims

1. Method of improving a performance parameter in a horse, wherein said performance parameter is chosen from increase in lean muscle mass, reduction in lactic acid, increase in workload tolerance, and faster recuperation after workload,
wherein said method comprises administering to said horse a composition formulated to provide 0.1 to 10 mg/150 kg subject/day of n-glycolylneuraminic acid.

2. Method according to claim 1, wherein said n-glycolylneuraminic acid is derived from natural sources.

3. Method according to claim 2, wherein said natural source is a non-animal or non-mammalian source.

4. Method according to Claim 1, wherein said n-glycolylneuraminic acid is chemically synthesized.

5. Method according to any one of claims 1 to 4, further comprising administering at least one composition selected from the group consisting of Avena sativa extract, Fabaceae extract, and Holothuroidea echinodermata extract.

6. Method according to any of claims 1 to 5, wherein the composition further comprises one or more mineral supplements selected from the group consisting of Fe, Zn, Cu and Mn.

7. Method according to any of claims 1 to 6, wherein said composition is formulated to provide a daily dosage of said n-glycolylneuraminic acid, selected from the group consisting of 0.3 to 5 mg/150 kg subject/day, and 0.5 to 2 mg/150 kg subject/day.

8. Method according to any of claims 1 to 7, wherein said composition is formulated as a powder, tablet, bolus, liquid or gel.

9. Method according to any of claims 1 to 8, wherein said composition is formulated for an administration mode selected from the group consisting of enteral administration, parenteral administration, oral administration, subcutaneous administration, intramuscular administration, and intravenous administration.

## Patentansprüche

1. Verfahren zur Verbesserung eines Leistungsparameters in einem Pferd, wobei der Leistungsparameter aus einer Erhöhung der Muskelmasse, einer Verminderung von Milchsäure, einer Erhöhung der Arbeitslasttoleranz und einer schnelleren Erholung nach Arbeitsbelastung ausgewählt ist,
wobei das Verfahren die Verabreichung einer zur Bereitstellung von 0,1 bis 10 mg/150 kg Subjekt/Tag n-Glycolylneuraminsäure formulierten Zusammensetzung an das Pferd umfasst.

2. Verfahren nach Anspruch 1, wobei die n-Glycolylneuraminsäure aus natürlichen Quellen stammt.

3. Verfahren nach Anspruch 2, wobei es sich bei der natürlichen Quelle um eine Nicht-Tier- oder Nicht-Säugetier-Quelle handelt.

4. Verfahren nach Anspruch 1, wobei die n-Glycolylneuraminsäure chemisch synthetisiert ist.

5. Verfahren nach einem der Ansprüche 1 bis 4, weiterhin umfassend die Verabreichung mindestens einer Zusammensetzung ausgewählt aus der aus Avena sativa-Extrakt, Fabaceae-Extrakt und Holothuroidea echinodermata-Extrakt bestehenden Gruppe.

6. Verfahren nach einem der Ansprüche 1 bis 5, wobei die Zusammensetzung weiterhin ein oder mehrere aus der aus Fe, Zn, Cu und Mn bestehenden Gruppe ausgewählte Mineralstoffe umfasst.

7. Verfahren nach einem der Ansprüche 1 bis 6, wobei die Zusammensetzung zur Bereitstellung einer Tagesdosis an n-Glycolylneuraminsäure ausgewählt aus der aus 0,3 bis 5 mg/150 kg Subjekt/Tag und 0,5 bis 2 mg/150 kg Subjekt/Tag bestehenden Gruppe formuliert ist.

8. Verfahren nach einem der Ansprüche 1 bis 7, wobei die Zusammensetzung als ein Pulver, eine Tablette, ein Bolus, eine Flüssigkeit oder ein Gel formuliert ist.

9. Verfahren nach einem der Ansprüche 1 bis 8, wobei die Zusammensetzung für einen aus der aus enteraler Verabreichung, parenteraler Verabreichung, oraler Verabreichung, subkutaner Verabreichung, intramuskulärer Verabreichung und intravenöser Verabreichung bestehenden Gruppe ausgewählten Verabreichungsmodus formuliert ist.

## Revendications

1. Procédé d'amélioration d'un paramètre de performance chez un cheval, où ledit paramètre de performance est choisi parmi l'augmentation de la masse musculaire maigre, la réduction de l'acide lactique, l'augmentation de la tolérance à la charge de travail et une récupération plus rapide après une charge de travail,
dans lequel ledit procédé comprend le fait d'administrer audit cheval une composition formulée pour fournir 0,1 à 10 mg/150 kg de sujet/jour d'acide n-glycolylneuraminique.

2. Procédé selon la revendication 1, dans lequel ledit acide n-glycolylneuraminique est dérivé de sources naturelles.

3. Procédé selon la revendication 2, dans lequel ladite source naturelle est une source non animale ou non mammifère.

4. Procédé selon la revendication 1, dans lequel ledit acide n-glycolylneuraminique est synthétisé chimiquement.

5. Procédé selon l'une quelconque des revendications 1 à 4, comprenant en outre le fait d'administrer au moins une composition choisie dans le groupe constitué par un extrait d'Avena sativa, un extrait de Fabaceae et un extrait d'Holothuroidea echinodermata.

6. Procédé selon l'une des revendications 1 à 5, dans lequel la composition comprend en outre un ou plusieurs complément(s) minéral/minéraux choisi(s) dans le groupe constitué par Fe, Zn, Cu et Mn.

7. Procédé selon l'une des revendications 1 à 6, dans lequel ladite composition est formulée pour fournir une dose quotidienne dudit acide n-glycolylneuraminique, choisie dans le groupe constitué par 0,3 à 5 mg/150 kg de sujet/jour, et 0,5 à 2 mg/150 kg de sujet/jour.

8. Procédé selon l'une des revendications 1 à 7, dans lequel ladite composition est formulée sous forme de poudre, de comprimé, de bolus, de liquide ou de gel.

9. Procédé selon l'une des revendications 1 à 8, dans lequel ladite composition est formulée pour un mode d'administration choisi dans le groupe constitué par l'administration entérale, l'administration parentérale, l'administration orale, l'administration sous-cutanée, l'administration intramusculaire et l'administration intraveineuse.
